# EUROPEAN PATENT SPECIFICATION

(11) **EP 3 226 954 B1**
(45) Date of publication and mention of the grant of the patent: **01.01.2020**
(21) Application number: 15865761.9
(22) Date of filing: 30.11.2015
(51) Int. Cl.: A61M 25/00, A61M 25/06, A61B 17/34, A61B 90/00, A61M 5/31

(54) **DISTALLY ORIENTED NEEDLE OBTURATOR**
DISTAL ORIENTIERTER NADELOBTURATOR
OBTURATEUR D'AIGUILLE ORIENTÉ DE MANIÈRE DISTALE

(30) Priority: 02.12.2014 US 201462086544 P
(43) Date of publication of application: 11.10.2017
(73) Proprietor: Merit Medical Systems, Inc., South Jordan, UT 84095 (US)
(72) Inventor: CAVILLA, Matt, Salt Lake City, Utah 84109 (US); CARLSTROM, Steve, Salt Lake City, Utah 84124 (US)
(74) Representative: Ricker, Mathias
(86) International application number: PCT/US2015/062986
(87) International publication number: WO 2016/089767

(56) References cited:
- GB-A- 584 600
- US-A- 1 336 730
- US-A- 3 405 713
- US-A- 5 342 383
- US-A1- 2002 072 712
- US-A1- 2004 097 881
- US-B1- 7 056 314
- US-B2- 8 100 859

## Description

### TECHNICAL FIELD

The present disclosure relates generally to medical devices for facilitating use and placement of elongate medical tubes, such as pigtail catheters or other drainage catheters, in a body cavity or tissue of a patient, and in particular, to safety mechanisms for protecting the medical device and the practitioners using the medical device. An obturator for facilitating straightening of an elongate drainage catheter is an exemplary embodiment of such a device. The prior art document US 1 336 730 A (D1) discloses a syringe system, which serves to allow perfect sterile injection into the human body. The mandrel (stylet) disclosed in D1 serves to pierce a lid of metal used to isolate a liquid for injecting into the human body from the actual syringe used for injection.

### BRIEF DESCRIPTION OF THE DRAWINGS

The embodiments disclosed herein will become more fully apparent from the following description and appended claims, taken in conjunction with the accompanying drawings. While various aspects of the embodiments are presented in drawings, the drawings depict only typical embodiments, which will be described with additional specificity and detail through use of the accompanying drawings in which:
FIG. 1 is a perspective view of an embodiment of an obturator device.
FIG. 2 is an exploded view of the obturator device of FIG. 1.
FIG. 3 is a perspective view of a needle insertion device, including the obturator device of FIG. 1.
FIG. 4 is a cross-sectional view taken along section 4-4 of the needle insertion device of FIG. 3.
FIG. 4A is an enlarged view of section 4A of FIG. 4.
FIG. 5A is a side view of the obturator device of FIG. 1.
FIG. 5B is a side view of another embodiment of an obturator device.
FIG. 5C is a side view of yet another embodiment of an obturator device.
FIG. 6 is a perspective view of the needle insertion device of FIG. 3 positioned within a pigtail drainage catheter.
FIG. 7 is a partially exploded view of the needle insertion device and pigtail drainage catheter of FIG. 6.

### DETAILED DESCRIPTION

The present disclosure is directed to an obturator device configured to be coupled with and protect a needle of a needle insertion device, and to facilitate preparation and insertion of elongate medical tubes, such as pigtail catheters, in a body cavity or tissue of a patient. According to one embodiment of the present disclosure, the obturator device includes a jacket with a tapered proximal end configured to match the tapered end of a needle bevel. When the needle insertion device is in an assembled configuration, the jacket rests against and mates with the needle bevel to protect the bevel from wear, and to avoid inadvertently puncturing or piercing the elongate medical tube when the needle insertion device is used during a medical procedure. The jacket also serves to protect the practitioner from possible injury, such as by accidentally pricking himself or another with the exposed needle. The jacket and needle may be secured together via a sleeve, such as a heat-shrink tube, that encircles a mating junction of the jacket and needle. Additional details of this and other embodiments of the obturator device are described in further detail below with specific reference to the figures.

It will be appreciated by one of skill in the art having the benefit of this disclosure, that various features of the devices disclosed herein are sometimes grouped together in a single embodiment, figure, or description thereof for the purpose of streamlining the disclosure. Many of these features may be used alone and/or in combination with one another. It will further be appreciated by one of skill in the art having the benefit of this disclosure, that many of the features disclosed herein may be used in conjunction with other assemblies presently known or hereafter developed.

Embodiments may be understood by reference to the drawings, wherein like parts are designated by like numerals throughout. It will be readily understood that the components of the present disclosure, as generally described and illustrated in the drawings herein, could be arranged and designed in a wide variety of different configurations. The invention is defined by the patent claims. In some cases, well-known structures, materials, or operations are not shown or described in detail. While the various aspects of the embodiments are presented in drawings, the drawings are not necessarily drawn to scale unless specifically indicated.

The phrases "connected to," "coupled to," and "in communication with" are used in an expansive sense in the present disclosure and refer to any form of interaction between two or more structures, entities or components, including but not limited to mechanical, fluidic, electrical, or magnetic interaction. Two components may be coupled to each other even though they are not in direct contact with each other. For example, two components may be coupled to each other through an intermediate component.

The terms "proximal" and "distal" are used to establish a frame of reference and refer to opposite ends of a medical device, including the devices disclosed herein. As used herein, the "proximal" portion or end of a medical device is the portion nearest a practitioner during use, while the "distal" portion or end of the medical device is opposite the proximal portion (e.g., the distal portion of the medical device is furthest away the practitioner during use). For example, the proximal end of a needle assembly comprises the end nearest the practitioner during normal use, such as a hub end of the assembly, with the distal end comprising the opposite end, such as a sharpened bevel end. For other components of a needle assembly, such as an oburator or catheter, the coordinate system is based on the orientation of each component with respect to the needle when the components are in an assembled configuration. The coordinate system does not change if the components are disassembled (for example if the obturator is removed from the assembly), even if the position of that component changes with respect to the practitioner. For example a practitioner may grasp the distal end of an obturator coupled to a needle in order to remove the obturator from the assembly and the coordinate system does not change, notwithstanding the distal end of the obturator may temporarily be closer to the practitioner.

It should be understood that while the present disclosure may refer to a pigtail catheter as an example elongate medical device, a variety of elongate medical devices may be utilized with the obturator device and/or needle insertion device of the present disclosure. For example, elongate medical devices as described herein include, but are not limited to, epidural catheters, drainage catheters, interventional tools, and guidewires. Thus, specific disclosure below referencing specific elongate medical devices, such as pigtail catheters, should be understood to analogously apply to other elongate medical devices.

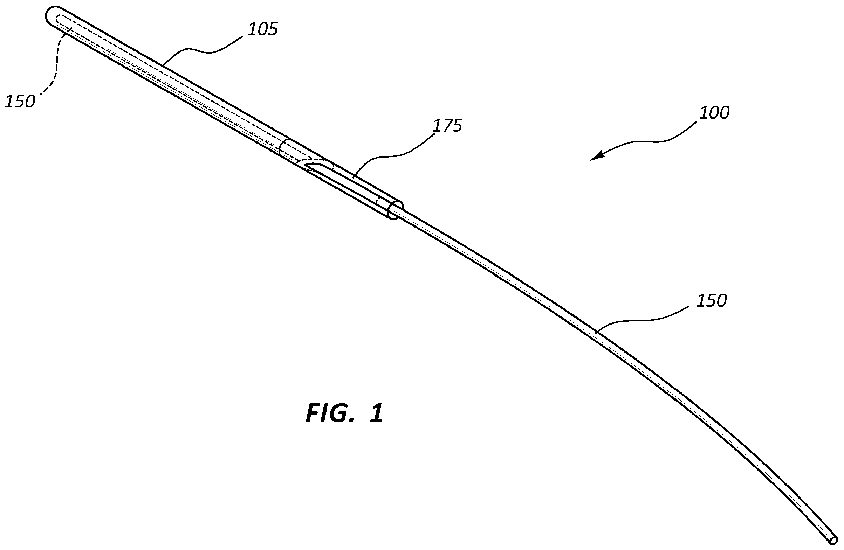

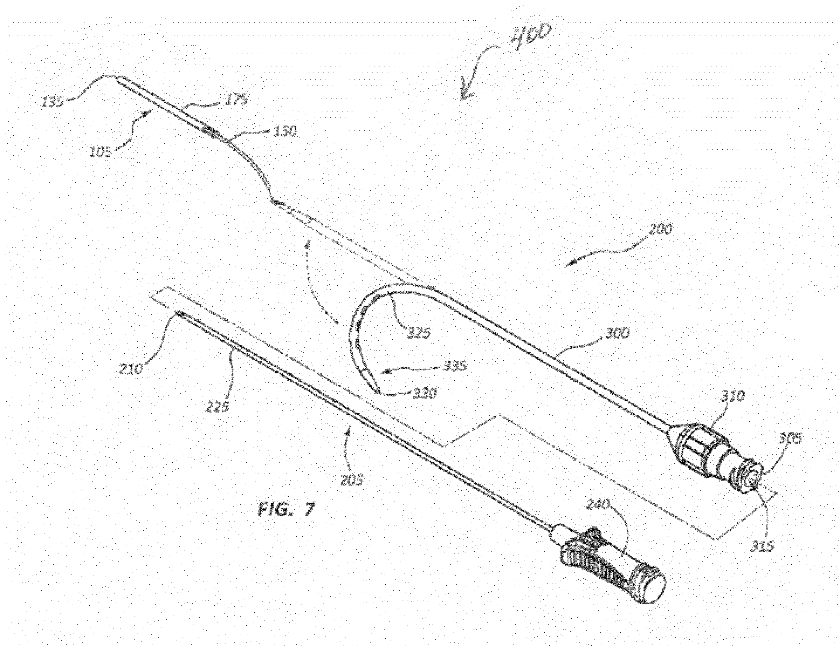

FIGS. 1-7 illustrate various detailed views of a needle insertion device 200 used to straighten a pigtail catheter (or other elongate medical device) 300 and to facilitate insertion of the catheter 300 into a patient for drainage of a body cavity or tissue. As mentioned previously, the needle insertion device 200 includes an obturator device 100 mated with or coupled to a distal end of a needle 205 to facilitate insertion and maneuvering of the needle insertion device 200 when straightening the catheter 300, and to protect against the bevel 210 of the needle 205 inadvertently puncturing or catching the interior walls (not shown) of the catheter 300. The obturator device 100 may also serve to protect the practitioner or others handling the needle 205 from accidental injury.

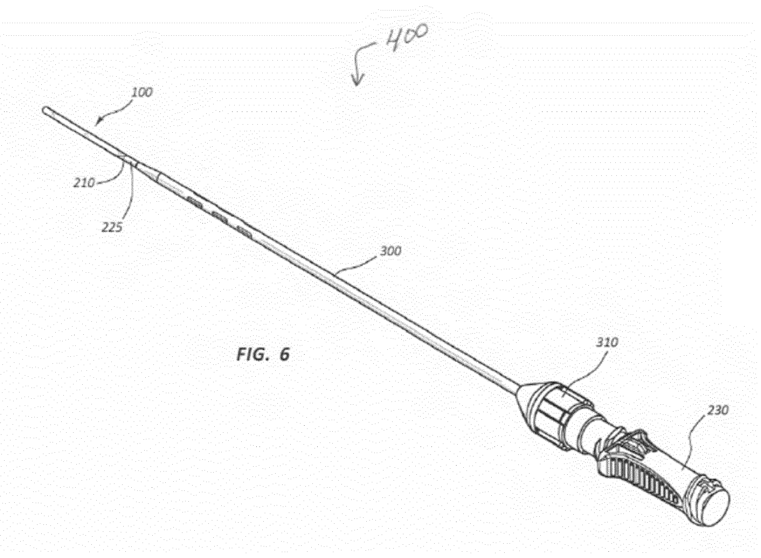

As is further described in detail below, in one example medical procedure, the needle insertion device 200 is inserted into a cavity or lumen 315 of the pigtail catheter 300. As the needle insertion device 200 is advanced inside the pigtail catheter 300, the obturator device 100 rides against the inside walls of the catheter 300 to smoothly guide the needle insertion device 200 through the lumen 315 and straighten out a coiled portion 325 of the pigtail catheter 300. When the pigtail catheter 300 is in a fully straightened configuration (see FIG. 6), the jacket 105 and the needle bevel 210 both extend from an opening 330 of the pigtail catheter 300. In this configuration, the obturator device 100 is removed to expose the bevel 210. The bevel 210 may thereafter be used to puncture the patient body and advanced until the desired body cavity or tissue for drainage is reached. Thereafter, the needle 205 is retracted from within the pigtail catheter 300 to complete the medical procedure. Additional details of the obturator device 100, the needle insertion device 200, and example assemblies of these devices are described in further detail below with reference to the embodiments illustrated in the figures.

FIGS. 1-4A collectively illustrate an example embodiment of the needle insertion device 200 and its components. As mentioned previously, the needle insertion device 200 includes an obturator device 100 coupled to a needle 205. The following section proceeds with a detailed discussion of the components of the obturator device 100, followed by a discussion of an example configuration of the needle insertion device 200. Thereafter, with reference to FIGS. 6-7, follows a discussion of an example use of the needle insertion device 200 with a catheter 300.

With particular reference to FIGS. 1-2, the obturator device 100 includes a jacket 105 having a proximal end 110 and a distal end 115. In the illustrated embodiment, the jacket 105 is a hollow and generally tubular structure including a cavity or passageway 120 that extends between the proximal end 110 and the distal end 115. In other embodiments the jacket 105 may comprise a solid member. In the illustrated configuration, the proximal end 110 of the jacket 105 includes an opening 125 formed on a face 130, and the distal end 115 includes a closed end 135 that may be rounded or radiused to provide a smooth, contoured surface that bears against and rides along the interior wall of the catheter 300 to facilitate advancement of the needle insertion device 200 as is described in further detail below with respect to FIGS. 6-7. In other embodiments, the jacket 105 may instead be an open tube having an opening at the distal end 115.

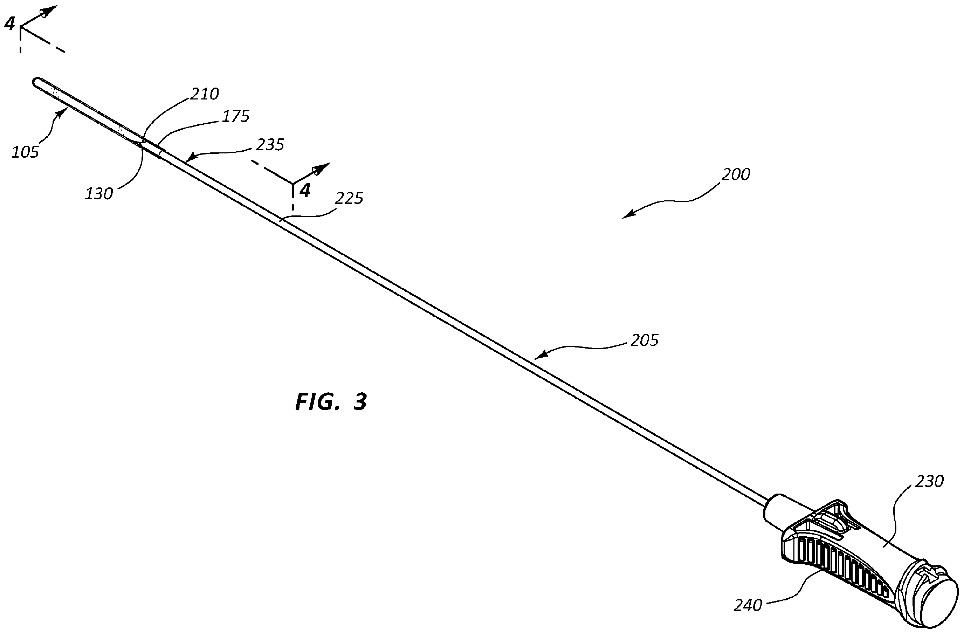

The face 130 at the proximal end 110 of the jacket 105 is shaped to correspond with or mirror the contours of the bevel 210 of the needle 205 so that the face 130 rests flush against the bevel 210 when the needle insertion device 200 is assembled. In other words, the shape and configuration of the needle bevel 210 may dictate the shape and configuration of the face 130 of the jacket 105. For example, in one conventional design, the needle bevel 210 is a single, angled surface that forms a tip at one end of the needle shaft 225 (see FIG. 3) and the face 130 may have a correspondingly shaped surface. The following description proceeds using the illustrated bevel 210 for illustration purposes, but it should be understood that in other embodiments, the needle bevel 210 and face 130 of the jacket 105 may have any one of a number of configurations. For example, in some embodiments, the needle may be an epidural needle for inserting an epidural catheter and may include any one of a variety of point styles, such as a Crawford needle, a Tuohy needle, a Hustead needle, a Weiss needle, or a Sprotte Spezial needle. As noted previously, the face 130 of the jacket 105 may have a suitable shape to match the unique bevel shape for any of these (or other) needles so that the jacket 105 sits flush against the bevel as described previously. Thus, regardless of the shape of the distal end of a needle, a jacket may be configured to mate with and/or match that surface. In some embodiments, a mating surface of the jacket thus forms the negative surface of the sharp portion of a needle surface.

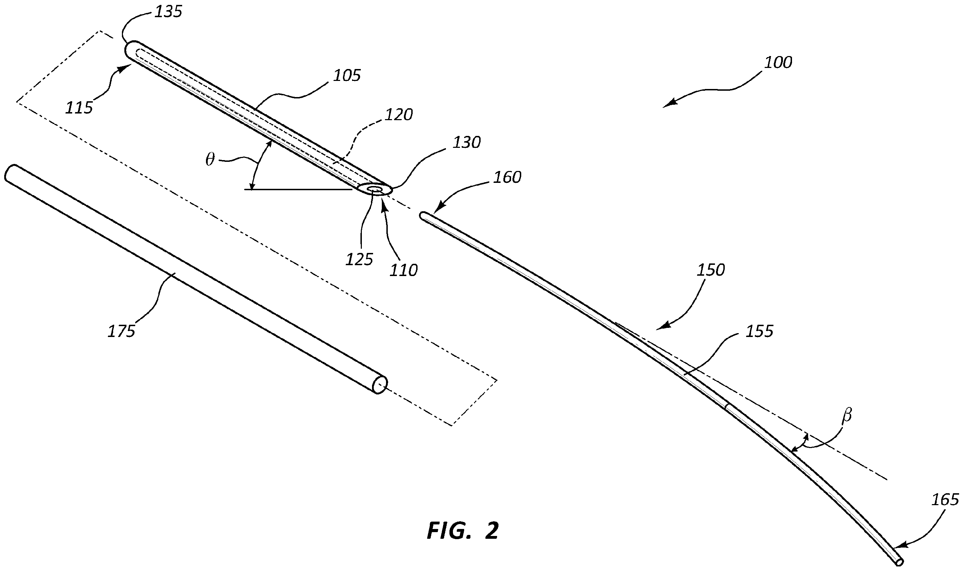

With particular reference to FIG. 2, the face 130 may be a sloped or beveled surface formed at an angle θ substantially equal to the angle of the bevel 210 so that the face 130 sits substantially flush against the needle bevel 210 when the jacket 105 is mated with the needle 205. For example, in some embodiments, the face 130 may be beveled at an angle θ ranging from between 0° and 90° to match the angle of the bevel 210. In other embodiments, the face 130 may be beveled at an angle θ ranging from between 10° and 60°. In one embodiment, the needle 205 may have a standard or A-bevel point style having a sloped surface ranging from between 12° to 15°. In such embodiments, the angle θ of the face 130 may also range from 12° to 15° to match the bevel so that jacket 105 sits flush against the bevel 210 with none or minimal rough edges.

The jacket 105 may be manufactured or molded as a single, unitary body of a flexible, polymeric or synthetic material to minimize potential wear of the bevel 205 such as may occur from frictional movement or rubbing together between the jacket 105 and the bevel 205. For example, in some embodiments, the jacket 105 may be molded from polyethylene (PE), polyethylene terephthalate (PET), polyvinyl chloride (PVC), polypropylene (PP), or other suitable plastic material to minimize wear of the bevel 205.

With particular reference to FIG. 2, the obturator device 100 further includes a mandrel 150 having an elongated, tubular body 155. The mandrel 150 may be composed or manufactured of a rigid material, such as stainless steel, metals, alloys, engineering plastics, or other suitable rigid materials. In some embodiments, the body 155 includes a substantially straight distal portion 160. Further, the proximal portion 165 of the mandrel 150 is arcuate.

In the illustrated configuration, the distal portion 160 of the mandrel 150 is seated and retained within the cavity 120 of the jacket 105, and the arcuate proximal portion 165 extends outwardly from the proximal end 110 of the jacket 105 in a longitudinal direction. In other embodiments the mandrel 150 may be coupled to the jacket 105 in a variety of ways. For example, the jacket 105 may comprise a solid member and the mandrel 150 may be forced into the jacket 105 to couple the components. In some such embodiments, the mandrel 150 may be heated to faciltate insertion and bonding. Other methods of coupling, including use of adhesive, other interference or friction fits, and use of radial restrains or other fasteners are within the scope of this disclosure.

In some embodiments, for example, the mandrel 150 may be retained in position within the jacket 105 by a frictional force between the interior walls of the jacket 105 and the outer surface of the mandrel 150. For example, the mandrel 150 may be machined or manufactured with an outer diameter dimensioned in relation to the diameter of the cavity 120 to create an interference or frictional fit between the mandrel 150 and the jacket 105. In such an arrangement, the mandrel 150 may be inserted into the cavity 120 of the jacket 150 and retained in position by frictional forces. In other embodiments, the mandrel 150 may instead be rigidly or adhesively coupled to the interior walls of the jacket 105 to further secure and prevent inadvertent removal of the mandrel 150.

The arcuate proximal portion 165 of the mandrel 150 may be configured to maintain the mandrel 150 within a needle lumen. As is explained in further detail below with respect to FIG. 3, the arcuate proximal portion 165 of the mandrel 150 may act as a spring or biasing force against an interior wall 220 of the needle shaft 225 to help retain the obturator device 100 in position against the needle 205. The arcuate portion 165 of the mandrel 150 may be angled at any suitable angle β to allow for the curved portion 165 to slide into the needle lumen 215 and bear against the interior walls 220 of the needle shaft 225. For example, in some embodiments, the angle β may range from between 10° or 20°, such as of approximately 15°. In other embodiments, the angle β may be smaller or larger depending on the gauge of the needle and the diameter of the needle shaft.

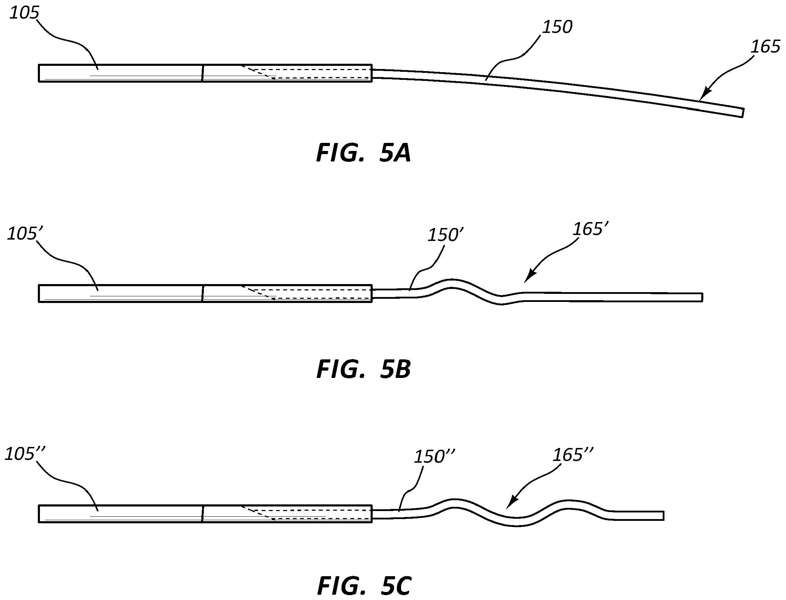

The arcuate portion 165 of the mandrel 150 is also shown in the side view of the obtruator device of FIG. 5A. The jacket 105 is also shown in this view. Furthermore, FIGS. 5B and 5C are side views of two alternative embodiments of obturator devices, each having a jacket 105', 105" and a mandrel 150' and 150". In the embodiment of FIG. 5B the mandrel 150' forms an arcute portion 165' comprising a single wave or bend. This single bend arcuate portion 165' may provide a spring or biasing force when partially compressed by an interior wall of a needle shaft, thus providing a force to help retain the obturator device within a needle. Mandrels with single wave bends, multiple wave bends, partial wave bends, and other shapes are within the scope of this disclosure. For example, in the embodiment of FIG. 5C, the mandrel 150" comprises an arcuate portion 165" comprising two waves or bends.

Regardless of the form or shape of the arcuate portion 165, 165', 165" the mandrel may thus provide a spring force to help maintain the obturator within a needle. In some embodiments the arcuate portion 165, 165', 165" may be configured to provide resistance to withdrawal of the obturator from a needle such that the withdrawal force is between 0.10 lbs and 2.0 lbs, including between 0.10 lbs and 1.8 lbs, between 0.5 lbs and 1.5 lbs, forces of less than 2 lbs, less than 1.75 lbs, less than 1.5 lbs, less than 1 lbs, less than 0.5 lbs, or less than 0.25 lbs.

With particular reference to FIG. 3, in the illustrated configuration, the distal portion 160 of the mandrel 150 is seated within the cavity 120 of the jacket 105 and spaced apart or offset from the distal end 115 of the jacket 105 to allow for some flexibility of the distal end 115 (or rounded end 135) when the distal end 115 rides along the interior walls of the pigtail catheter 300. In other embodiments, the distal portion 160 of the mandrel 150 may instead contact an interior portion of the rounded end 135 so that the mandrel 150 extends along the entire length of the cavity 120.

The obturator device 100 further includes a sleeve 175 encircling at least a portion of the jacket 105. In some embodiments, the sleeve 175 may extend to and wrap around the distal end 115 of the jacket 105 to further protect the distal end 115 and facilitate entry and maneuvering of the needle insertion device 200 when inserted into the pigtail catheter 300. The sleeve 175 may have an inner diameter that is larger than the outer diameter of the jacket 105 to allow the sleeve 175 to slide over and sit against the jacket 105. The sleeve 175 extends beyond the proximal end 110 and face 130 of the jacket 105 so that when the needle insertion device 200 is assembled, the sleeve 175 helps form a tight seal at the mating junction between the face 130 of the jacket 105 and the bevel 210 of the needle 205. Additional details of the assembled configuration are described below with reference to FIGS. 3-4A.

In some embodiments, the sleeve 175 may be a heat-shrink tube or other shrinkable plastic tube that shrinks when heat is applied to wrap tightly around the jacket 105 and is mechanically held in place by its tight fit. The heat-shrink tube may be adhesive-lined to form a secure seal around the jacket 105 and the bevel 210 (and portion of the needle shaft 225) to prevent moisture, debris, dust, or other foreign materials from entering the lumen 215. In other embodiments, the sleeve 175 may instead be rubber, neoprene, or other suitable material that may be fitted around the jacket 105 and bevel 210. In such embodiments, the sleeve 175 may be retained in position via a mechanical grip of the sleeve 175 with the jacket 105 and needle shaft 225.

Embodiments wherein the obturator device 100 is secured to a needle only by friction between the sleeve 175 and the needle are within the scope of this disclosure, as are embodiments wherein the entire securement force is provided by an arcuate bend in the mandrel 150 (such as arcuate portion 165 of FIG. 5A). Embodiments wherein any combination of these forces, and/or additional forces, couple the obturator device 100 to a needle are also within the scope of this disclosure.

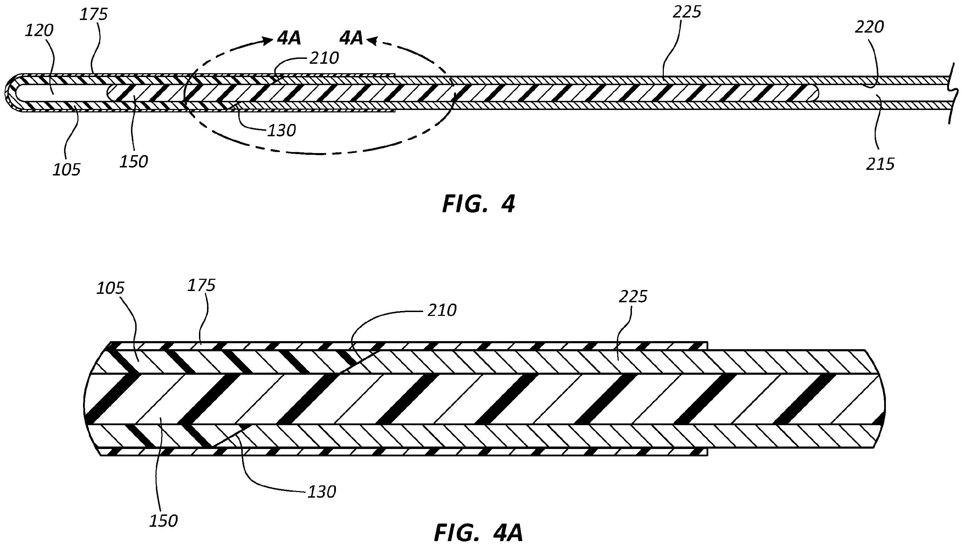

With reference to FIGS. 3-4A, the following section describes additional details of the needle insertion device 200 and an example assembly and coupling of the obturator device 100 with the needle 205. With particular reference to the cross-section views of FIGS. 4 and 4A, in an example assembly, the proximal end 110 of the jacket 105 is generally aligned with the bevel 210 of the needle 205 along a longitudinal direction, with the arcuate proximal portion 165 of the mandrel 150 facing the bevel 210. Thereafter, the mandrel 150 is inserted into the needle lumen 215 and advanced toward the proximal end 235 of the needle 205, with the arcuate proximal portion 165 riding and bearing against an interior wall 220 of the needle shaft 225. As the mandrel 150 is advanced, the jacket 105 may be repositioned or rotated to align the face 130 of the jacket 105 relative to the bevel 210 so that the face 130 generally corresponds with and mirrors the bevel 210. Once the face 130 and bevel 210 are aligned, the mandrel 150 is fully inserted into the needle lumen 215. The outer diameter of the jacket 105 may be equal or substantially equal to the outer diameter of the needle shaft 225 so that when the face 130 abuts the bevel 210, the jacket 105 is substantially flush with needle shaft 225 at the mating junction to minimize or eliminate catching or snagging of the pigtail catheter 300 during the medical procedure.

As mentioned previously, the arcuate proximal end 165 of the mandrel 150 bears against the internal wall 220 of the needle shaft 225 to retain the obturator device 100 in position. The diameter of the cavity 120 of the jacket 105 may be equal or substantially equal to the diameter of the needle lumen 215 to minimize slop or backlash of the mandrel 150 within the cavity 120 and/or the lumen 215 when the needle insertion device 200 is assembled.

Once the jacket 105 is positioned against the bevel 210, the sleeve 175 may be positioned over the jacket 105 from the distal end 115 and advanced toward the mating junction between the jacket 105 and the bevel 210. To help further secure the jacket 105 to the needle 205 and to minimize any rough edges that may be present at the mating junction, the sleeve 175 extends over the mating junction and at least onto a portion of the needle shaft 225. In embodiments where the sleeve 175 is a heat-shrink tube, heat may be applied to shrink the sleeve 175 around the jacket 105 and the needle shaft 225 to secure the components of the needle insertion device 200.

It should be understood that in other embodiments, the sleeve 175 may be positioned around the jacket 105 prior to inserting the mandrel 150 into the lumen 215. In such embodiments, the sleeve 175 may extend over and beyond the proximal end 110 of the jacket 105 and serve as a guide for mating the jacket 105 with the bevel 210. The sleeve 175 may also help protect a person assembling the needle insertion device 200 from inadvertent injury. In this configuration, the mandrel 150 is inserted into the lumen 215 in a similar manner as described previously and advanced until the needle bevel 210 and at least a portion of the shaft 225 are adjacent the sleeve 175. At this point, the bevel 210 and the shaft 225 are inserted into the sleeve 175 and advanced toward the face 130 of the jacket 105. Once the face 130 is mated with the bevel 210, then heat may be applied to shrink the sleeve 175 and secure the mating junction as described previously.

In its assembled configuration, the needle insertion device 200 may be inserted into a pigtail catheter 300 (or other similar drainage catheter or medical device) to straighten the catheter 300 and prepare it for insertion into a patient body or cavity. The following section describes an example embodiment of a device assembly 400 with particular reference to FIGS. 6-7. As illustrated in FIG. 6, the needle insertion device 200 includes a needle hub 230 on a proximal end 235. The needle hub 230 may include knurled or grooved section 240 to facilitate gripping and handling of the needle insertion device 200 by a practitioner during a medical procedure.

In an example embodiment of the device assembly 400, the needle insertion device 200 is aligned with an opening 305 on a catheter hub 310 of the catheter 300, with the distal end 115 of the jacket 105 facing the catheter opening 305. The distal end 115 of the jacket 105 is inserted into the opening 305 and advanced into a catheter lumen 315. As the needle insertion device 200 is advanced, the radiused end 125 of the jacket 105 (and the sleeve 175) rides against an internal wall (not shown) of the catheter 300. As the needle insertion device 200 is pushed further into the lumen 315, the jacket 105 straightens a coiled portion 325 of the pigtail catheter 300. The jacket 105 and the sleeve 175 protect against any inadvertent piercing or puncturing of the pigtail catheter 300 that may be caused by the needle bevel 210. The position of the jacket 105 against the bevel 210 and/or the position of the sleeve 175 around the bevel 210 may thus isolate the bevel 210 from interaction with the pigtail catheter 300.

The needle insertion device 200 is advanced into the catheter lumen 315 until the jacket 105 extends beyond an opening 330 on a distal portion 335 of the pigtail catheter 300. When the needle insertion device 200 is fully inserted, the pigtail catheter 300 is in a straightened configuration (as shown in FIG. 6), with at least a portion of the jacket 105 exposed and extending beyond the opening 330 on the distal portion 335 of the catheter 300. In this configuration, at least a portion of the bevel 210 of the needle 205 and a portion of the shaft 225 also protrude from the opening 330, with a substantial portion of the needle shaft 225 remaining inside the catheter lumen 315.

Prior to inserting the catheter 300 into a patient, the obturator device 100 is removed from the distal portion 335 of the catheter 300 to expose the bevel 210. In some embodiments, the obturator device 100 may be pulled distally with sufficient force to break the seal formed by the heat-shrink sleeve 175 and overcome its mechanical grip on the needle bevel 210 and shaft 225. When the obturator device 100 is removed, the mandrel 150 is also removed from within the needle lumen 215.

Accordingly, the obturator device 100 may be removed by distally displacing the obturator device 100 with respect to the needle 205. Similarly, when assembled, the obturator device 100 may simultaneously be distally displaced with respect to the catheter 300 as the obturator device 100 is removed. In the illustrated assembly, the needle 205 is removed from the catheter 300 by proximally displacing the needle 205 (for example through manipulation of the needle hub 240) with respect to the catheter 300. Thus, removal of the obturator device 100 may be completed by displacing the obturator device 100 from the catheter 300 in the opposite direction from which the needle 205 is displaced to remove the needle 205 from the catheter.

After the obturator device 100 is removed and the bevel 210 exposed, the bevel 210 may be used to pierce the patient's body and advanced together with the pigtail catheter 300 to a desired body cavity or tissue. Once the pigtail catheter 300 is positioned at its desired location, the needle shaft 225 is retracted from the lumen 315 of the catheter 300 and removed via the opening 330 on the catheter hub 310, leaving the catheter 300 in position within the body cavity or tissue.

References to approximations are made throughout this specification, such as by use of the term "substantially." For each such reference, it is to be understood that, in some embodiments, the value, feature, or characteristic may be specified without approximation. For example, where qualifiers such as "about" and "substantially" are used, these terms include within their scope the qualified words in the absence of their qualifiers. For example, where the term "substantially straight" is recited with respect to a feature, it is understood that in further embodiments, the feature can have a precisely straight configuration.

Reference throughout this specification to "an embodiment" or "the embodiment" means that a particular feature, structure, or characteristic described in connection with that embodiment is included in at least one embodiment. Thus, the quoted phrases, or variations thereof, as recited throughout this specification are not necessarily all referring to the same embodiment.

Similarly, it should be appreciated that in the above description of embodiments, various features are sometimes grouped together in a single embodiment, figure, or description thereof for the purpose of streamlining the disclosure. This method of disclosure, however, is not to be interpreted as reflecting an intention that any claim requires more features than those expressly recited in that claim. Rather, as the following claims reflect, inventive aspects lie in a combination of fewer than all features of any single foregoing disclosed embodiment.

The claims following this written disclosure are hereby expressly incorporated into the present written disclosure, with each claim standing on its own as a separate embodiment. This disclosure includes all permutations of the independent claims with their dependent claims. Moreover, additional embodiments capable of derivation from the independent and dependent claims that follow are also expressly incorporated into the present written description. The invention is defined by the patent claims.

## Claims

1. An obturator device (100) comprising:
a jacket (105) having a proximal portion and an opposite distal portion, the proximal portion having a contoured face (130);
an elongated mandrel (150) having a proximal portion (165) and an opposite distal portion (160), wherein the distal portion (160) of the mandrel (150) is coupled to the jacket (105), and wherein the proximal portion (165) of the elongated mandrel (150) extends proximally from the jacket (105); and
a sleeve (175) encircling at least a portion of the jacket (105) and extending beyond the proximal portion of the jacket (105); **characterized in that**,
the proximal portion (165) of the mandrel (150) is arcuate.

2. The obturator device of claim 1, wherein the contoured face (130) comprises a tapered surface configured to mate with a bevel (210) of a needle (205).

3. The obturator device of any one of claims 1-2, wherein the jacket (105) further includes a cavity (120) extending between the proximal portion and the distal portion and wherein the distal portion (160) of the mandrel (150) is seated and retained within the cavity (120) of the jacket (105).

4. The obturator device of claim 3, wherein the distal portion (160) of the mandrel (150) bears against an interior wall of the jacket (105) to retain the distal portion (160) of the mandrel (150) in position within the cavity (120) of the jacket (105).

5. The obturator device of any one of claims 1-4, wherein the distal portion (160) of the mandrel (150) is offset from the distal portion of the jacket (105).

6. The obturator device of any one of claims 1-5, wherein the distal portion of the jacket (105) includes a rounded face.

7. The obturator device of any one of claims 1-6, wherein the distal portion of the jacket (105) includes a closed end (135).

8. The obturator device of any one of claims 1-7, wherein the sleeve (175) is a heat-shrink tube.

9. The obturator device of any one of claims 2-8, wherein the sleeve (175) includes a closed distal end and an open proximal end, the closed distal end encapsulating the distal portion of the jacket (105) and the open proximal end extending beyond the tapered face (130) of the proximal portion of the jacket (105).

10. The obturator device of claim 1, wherein the arcuate proximal portion (165) of the elongated mandrel (150) is configured to bear against a lumen (215) of a needle (205) to retain the mandrel (150) within the lumen (215).

11. The obturator device of claim 2, wherein the tapered surface (130) is angled between 10 degrees and 90 degrees.

12. The obturator device of claim 9, wherein the open proximal end of the sleeve (175) is configured to encircle at least a portion of the needle shaft (225).

13. A method of manipulating a needle assembly, the method comprising:
obtaining a needle (205) coupled to an obturator (100) according to claim 1 and a catheter (300), wherein,
a portion of the obturator (100) is disposed distally of a distal tip of the needle (205) and a portion of the needle (205) is disposed within a lumen (315) of the catheter (300); and removing the obturator (100) from the needle (205) and catheter (300) by displacing the obturator (100) distally with respect to the needle (205).

14. The method of claim 13, further comprising inserting the needle (205) and obturator (100) into the catheter lumen (315) prior to removing the obturator (100) from the needle (205) and catheter assembly.

## Patentansprüche

1. Obturatorvorrichtung (100), umfassend:
eine Ummantelung (105) mit einem proximalen Abschnitt und einem gegenüberliegenden distalen Abschnitt, wobei der proximale Abschnitt eine konturierte Fläche (130) aufweist;
einen länglichen Dorn (150) mit einem proximalen Abschnitt (165) und einem gegenüberliegenden distalen Abschnitt (160), wobei der distale Abschnitt (160) des Dorns (150) mit der Ummantelung (105) gekoppelt ist, und wobei sich der proximale Abschnitt (165) des länglichen Dorns (150) proximal von der Ummantelung (105) erstreckt; und
ein Schlauch (175), der mindestens einen Abschnitt der Ummantelung (105) umgibt und sich über den proximalen Abschnitt der Ummantelung (105) hinaus erstreckt;
**dadurch gekennzeichnet, dass**,
der proximale Abschnitt (165) des Dorns (150) bogenförmig ist.

2. Obturatorvorrichtung nach Anspruch 1, wobei die konturierte Fläche (130) eine abgeschrägte Oberfläche umfasst, die konfiguriert ist, um mit einer Fase (210) einer Nadel (205) zusammenzuwirken.

3. Obturatorvorrichtung nach einem der Ansprüche 1-2, wobei die Ummantelung (105) ferner einen Hohlraum (120) beinhaltet, der sich zwischen dem proximalen Abschnitt und dem distalen Abschnitt erstreckt, und wobei der distale Abschnitt (160) des Dorns (150) in dem Hohlraum (120) der Hülle (105) sitzt und gehalten wird.

4. Obturatorvorrichtung nach Anspruch 3, wobei der distale Abschnitt (160) des Dorns (150) an einer Innenwand der Ummantelung (105) anliegt, um den distalen Abschnitt (160) des Dorns (150) in Position innerhalb des Hohlraums (120) der Ummantelung (105) zu halten.

5. Obturatorvorrichtung nach einem der Ansprüche 1-4, wobei der distale Abschnitt (160) des Dorns (150) gegenüber dem distalen Abschnitt der Ummantelung (105) versetzt ist.

6. Obturatorvorrichtung nach einem der Ansprüche 1-5, wobei der distale Abschnitt der Ummantelung (105) eine abgerundete Fläche aufweist.

7. Obturatorvorrichtung nach einem der Ansprüche 1-6, wobei der distale Abschnitt der Ummantelung (105) ein geschlossenes Ende (135) beinhaltet.

8. Obturatorvorrichtung nach einem der Ansprüche 1-7, wobei der Schlauch (175) ein Wärme-Schrumpfschlauch ist.

9. Obturatorvorrichtung nach einem der Ansprüche 2-8, worin der Schlauch (175) ein geschlossenes distales Ende und ein offenes proximales Ende beinhaltet, wobei das geschlossene distale Ende den distalen Abschnitt der Ummantelung (105) und das offene proximale Ende über die abgeschrägte Fläche (130) des proximalen Abschnitts der Ummantelung (105) hinausragen.

10. Obturatorvorrichtung nach Anspruch 1, wobei der bogenförmige proximale Abschnitt (165) des länglichen Dorns (150) konfiguriert ist, um gegen ein Lumen (215) einer Nadel (205) zu drücken, um den Dorn (150) innerhalb des Lumens (215) zu halten.

11. Obturatorvorrichtung nach Anspruch 2, wobei die abgeschrägte Oberfläche (130) einen Winkel zwischen 10 Grad und 90 Grad bildet.

12. Obturatorvorrichtung nach Anspruch 9, wobei das offene proximale Ende des Schlauchs (175) konfiguriert ist, um mindestens einen Abschnitt der Nadelschafts (225) zu umschließen.

13. Verfahren zum Manipulieren einer Nadelanordnung, wobei das Verfahren umfasst:
Erhalten einer Nadel (205), die mit einem Obturator (100) nach Anspruch 1 und einem Katheter (300) gekoppelt ist, wobei
ein Abschnitt des Obturators (100) distal von einer distalen Spitze der Nadel (205) angeordnet ist und ein Abschnitt der Nadel (205) innerhalb eines Lumens (315) des Katheters (300) angeordnet ist und Entfernen des Obturators (100) von der Nadel (205) und dem Katheter (300) durch Verschieben des Obturators (100) distal in Bezug auf die Nadel (205).

14. Verfahren nach Anspruch 13, ferner umfassend das Einsetzen der Nadel (205) und des Obturators (100) in das Katheterlumen (315) vor dem Entfernen des Obturators (100) von der Nadel (205) und der Katheteranordnung.

## Revendications

1. Dispositif obturateur (100) comprenant :
une chemise (105) ayant une partie proximale et une partie distale opposée, la partie proximale ayant une face profilée (130) ;
un mandrin allongé (150) ayant une partie proximale (165) et une partie distale opposée (160), dans lequel la partie distale (160) du mandrin (150) est couplée à la chemise (105), et dans lequel la partie proximale (165) du mandrin allongé (150) s'étend de manière proximale depuis la chemise (105) ; et
un manchon (175) encerclant au moins une partie de la chemise (105) et s'étendant au-delà de la partie proximale de la chemise (105) ; **caractérisé en ce que**,
la partie proximale (165) du mandrin (150) est arquée.

2. Dispositif obturateur selon la revendication 1, dans lequel la face profilée (130) comprend une surface effilée configurée pour s'accoupler avec un biseau (210) d'une aiguille (205).

3. Dispositif obturateur selon l'une quelconque des revendications 1-2, dans lequel la chemise (105) inclut en outre une cavité (120) s'étendant entre la partie proximale et la partie distale et dans lequel la partie distale (160) du mandrin (150) est disposée et retenue dans la cavité (120) de la chemise (105).

4. Dispositif obturateur selon la revendication 3, dans lequel la partie distale (160) du mandrin (150) est en appui contre une paroi intérieure de la chemise (105) pour maintenir la partie distale (160) du mandrin (150) en position dans la cavité (120) de la chemise (105).

5. Dispositif obturateur selon l'une quelconque des revendications 1-4, dans lequel la partie distale (160) du mandrin (150) est décalée par rapport à la partie distale de la chemise (105).

6. Dispositif obturateur selon l'une quelconque des revendications 1-5, dans lequel la partie distale de la chemise (105) inclut une face arrondie.

7. Dispositif obturateur selon l'une quelconque des revendications 1-6, dans lequel la partie distale de la chemise (105) inclut une extrémité fermée (135).

8. Dispositif obturateur selon l'une quelconque des revendications 1-7, dans lequel le manchon (175) est un tube thermorétractable.

9. Dispositif obturateur selon l'une quelconque des revendications 2-8, dans lequel le manchon (175) inclut une extrémité distale fermée et une extrémité proximale ouverte, l'extrémité distale fermée encapsulant la partie distale de la chemise (105) et l'extrémité proximale ouverte s'étendant au-delà de la face effilée (130) de la partie proximale de la chemise (105).

10. Dispositif obturateur selon la revendication 1, dans lequel la partie proximale (165) arquée du mandrin allongé (150) est configurée pour être en appui contre une lumière (215) d'une aiguille (205) pour maintenir le mandrin (150) dans la lumière (215).

11. Dispositif obturateur selon la revendication 2, dans lequel la surface effilée (130) forme un angle entre 10 degrés et 90 degrés.

12. Dispositif obturateur selon la revendication 9, dans lequel l'extrémité proximale ouverte du manchon (175) est configurée pour encercler au moins une partie de la tige d'une aiguille (225).

13. Procédé de manipulation d'un dispositif aiguille, le procédé comprenant :
obtention d'une aiguille (205) couplée à un obturateur (100) selon la revendication 1 et un cathéter (300), dans lequel,
une partie de l'obturateur (100) est disposée de manière distale d'une pointe distale de l'aiguille (205) et une partie de l'aiguille (205) est disposée dans une lumière (315) du cathéter (300) ; et retrait de l'obturateur (100) de l'aiguille (205) et du cathéter (300) en déplaçant l'obturateur (100) de manière distale par rapport à l'aiguille (205).

14. Procédé selon la revendication 13, comprenant en outre l'insertion de l'aiguille (205) et de l'obturateur (100) dans la lumière du cathéter (315) avant le retrait de l'obturateur (100) de l'aiguille (205) et du dispositif cathéter.
